# EUROPEAN PATENT SPECIFICATION

(11) **EP 0 857 055 B1**
(45) Date of publication and mention of the grant of the patent: **18.12.2002**
(21) Application number: 96934544.6
(22) Date of filing: 04.10.1996
(51) Int. Cl.: A61K 7/32

(54) **UNDERARM COMPOSITIONS**
ACHSELZUSAMMENSETZUNGEN
COMPOSITIONS POUR LES AISSELLES

(30) Priority: 27.10.1995 GB 9521990
(43) Date of publication of application: 12.08.1998
(73) Proprietor: UNILEVER PLC, London EC4P 4BQ (GB); UNILEVER N.V., 3013 AL Rotterdam (NL)
(72) Inventor: CORREIA, Martinho, B-4000 Liege (BE); HAGAN, Desmond, Bernard, South Wirral, Cheshire L66 5NR (GB)
(74) Representative: Rots, Maria Johanna Francisca
(86) International application number: EP9604329
(87) International publication number: WO97016162

(56) References cited:
- EP-A- 0 312 270
- EP-A- 0 549 223
- EP-A- 0 707 845

## Description

The invention relates to novel cream underarm compositions which contain a structuring wax material. More particularly, the invention concerns antiperspirant and deodorant compositions for topical application to the human skin containing structuring wax.

It is known for underarm compositions for topical application to contain non-volatile silicone fluids such as polyorganosiloxanes which impart emolliency to the composition and can provide a masking effect to conceal solids present in the composition. Examples of such solids include antiperspirant actives. The efficacy of the composition is not seriously affected by the presence of the non-volatile silicone. An example of such compositions is to be found in EP 28853 (The Procter & Gamble Company).

However, many of the wax containing formulations of the prior art, whilst improving skin feel in some respects (e.g. reducing the wet sensation) can still result in visible deposits on the skin and fabrics.

In an attempt to overcome this problem EP135315 (The Mennen Company) describes a clay free antiperspirant product containing a volatile silicone and a gelling agent. However, the omission of the clay suspending agent can result in a product which is still unstable at high temperatures and over prolonged periods and which has an excessively low viscosity which can-be undesirable in cream formulations.

It is also known to include certain categories of alkyl siloxane waxes in underarm compositions. EP 549223 (Dow Corning) describes stick, roll on and spray underarm compositions containing certain long chain alkyl silicone waxes. The waxes are alleged to provide the formulations with desirable characteristics such as improved hardness, reduced whitening, improved skin feel and compatibility with other ingredients.

An object of the invention is to overcome the disadvantages of the formulations of the prior art.

According to the invention there is provided a substantially anhydrous underarm cream composition suitable for topical application to the human skin, comprising an antiperspirant and/or deodorant agent, a non-volatile masking agent, and a structuring wax having the general formula:

R - (SiMe₂-O-)ₓ SiMe₂R

where
- x =: 10-50
- Me =: CH₃
wherein, R is an alkyl group and the average terminal alkyl chain length is at least C30, characterized in that the composition has a cone penetration value of between approximately 10 mm and 30 mm when the cone has a diameter of 16.5 mm and a weight of 102.5 g.

Preferably, the composition comprises 3% to 20% by weight structuring wax. More preferably, the composition comprises 5 to 15% by weight structuring wax. Suitably, R is a C25 to C40 alkyl group.

Advantageously the underarm composition further comprises a non-volatile masking oil. Suitably, the masking oil is selected from the group comprising non-volatile silicones and polyolefins or mixtures thereof.

Preferably, the polyolefin is a polydecene.

Additionally, the underarm composition further comprises a volatile carrier fluid. Suitably, the volatile carrier fluid is a volatile silicone.

Advantageously, the antiperspirant agent is an inorganic and/or organic salt of aluminum and zirconium or a mixture thereof.

Suitably, the structuring wax is blended with alkyl ester siloxane waxes, insect and animal waxes, fatty acids, fatty alcohols, fatty acid esters and fatty acid amides and mixtures thereof.

In an alternative embodiment the invention also provides a substantially anhydrous cream antiperspirant composition comprising 5 to 30% by weight antiperspirant salt, 5% to 25% by weight of a non-volatile masking oil, 3% to 25% by weight of a structuring wax having the general formula:

R - (SiMe₂-o-)ₓ SiMe₂R

where
- x =: 10-50
- Me =: CH₃
- R: is an alkyl group
and the average terminal alkyl chain length is at least C30 and 20 to 87% by weight of a volatile carrier fluid, characterized in that the composition has a cone penetration value of between approximately 10 mm and 30 mm when the cone has a diameter of 16.5 mm and a weight of 102.5 g.

Generally, creams are defined in terms of cone penetration values. A typical cream can be described as having a cone penetration value of between approximately 10mm and 30mm where the cone has a diameter of 16.5mm and a weight of 102.5g.

Surprisingly, it has now been found that by selecting alkyl siloxane waxes that have specific alkyl group characteristics an underarm composition which has desirable viscosity characteristics and is stable for long periods and at high temperatures is obtained. Moreover, the composition has fewer of the undesirable characteristics such as poor skin feel and deposition previously associated with the previously described alkyl siloxane wax containing underarm compositions.

Typically used antiperspirant salts include inorganic and organic salts of aluminium and zirconium and mixtures thereof. Particularly preferred are the aluminium/zirconium salts of aluminium halides, aluminium hydroxyhalides, aluminium zirconium salts and mixtures thereof. Particularly preferred antiperspirant salts include activated aluminium chlorohydrate compounds as described in EP6739 (Unilever NV et al). Further antiperspirant actives are described in EP 28853. The contents of both these applications are incorporated herein by reference.

Any effective deodorant composition known in the art is suitable for incorporation into the composition e.g. sodium bicarbonate, zinc ricinoleate, other inorganic salts, short chain monohydric alcohols, polyhydric alcohols or compounds such as triclosan. The deodorants can be utilised alone or in conjunction with the antiperspirant active component where compatible.

The carrier fluid suitably comprises a volatile silicone material. Examples of such materials are cyclic or linear polydimethylsiloxanes. Preferred cyclic polydimethylsiloxanes have from 3 - 7 silicone atoms and a viscosity less than 10 mn²s⁻¹ (cSt) at 25°C. Preferred linear polydimethylsiloxanes have from 3 - 9 silicone atoms and a viscosity of less than 5 mm²s⁻¹ at 25°C. Preferred polydimethylsiloxanes are available from Dow Corning Corporation as Dow Corning 344 and 345. Preferably, if used in the composition, the volatile silicone is present at a level of 30 to 60% by weight, more preferably 40 to 60% by weight.

A non-volatile masking agent is also present in the formulation and preferably comprises a hydrocarbon polymer such as poly olefins.

The poly olefins are suitably hydrocarbon polymers, with the preferred ones being liquid at room temperature (i.e. 21°C). It is also highly preferred that the poly olefin in the composition has a relatively low viscosity. Preferably, the viscosity of the poly olefin hydrocarbon masking agent is less than about 40 cSt at 40°C, more preferably less than about 30 cSt at 40°C.

Preferably, the poly olefin comprises a poly alpha olefin. Preferred poly alpha olefins for use in compositions according to the invention are polydecenes, for example the Silkflo range of polydecenes, manufactured by Albermarle Corporation. Other preferred poly olefins for use in compositions according to the invention include polybutene, which is commercially available under the trade name Panalene L14E from Amoco, and polyisobutene, which can be obtained from Prespere under the trade name Permethyl.

As such, preferred poly olefins for use in compositions according to the invention may have monomer chain lengths in the region of 3-15 carbon atoms, and in their unpolymerised form may have one or two double bonds. Preferred poly olefin blends which are commercially available may conveniently contain a blend of various polymers, including dimers, trimers, and so on. Preferred materials for use in compositions according to the invention include Silkflo 362NF, Silkflo 364NF, and Silkflo 366NF, available from Albermarle Corporation.

When used in compositions according to the invention, the poly olefin hydrocarbons described help to confer to the composition surprisingly good sensory properties, including a surprising lack of greasiness after application, and where the composition is an antiperspirant composition, provide an enhanced degree of masking of any whiteness that may be left by the antiperspirant salt in the composition.

An advantage of using the poly olefin hydrocarbons is that they have been found not to interfere with the efficacy of any antiperspirant active salt in the composition to any major degree.

The non-volatile masking oil which can function as an emollient can also be a non-volatile silicone. The non-volatile silicone may be a polyalkyl siloxane, a polyalkaryl siloxane or a polyether siloxane copolymer. Preferred polyalkysiloxanes have viscosities ranging from 10 to 100,000 mm²s⁻¹ (cSt) at 25°C. Such siloxanes are available from the Dow Corning Corporation as the Dow Corning 200 series.

Suitable polyalkaryl siloxanes are the polymethylphenylsiloxanes having viscosities of 15 to 65mm²s⁻¹ (cSt) at 25°C. These siloxanes are available as the Dow Corning 556 fluid.

A suitable polyether siloxane is dimethyl polyoxyalkylene ether copolymer having a approximate viscosity of 1200 to 1500 mm²s⁻¹ (cSt) at 25°C e.g. a polysiloxane ethylene glycol ether copolymer.

The structuring waxes used are generally referred to as alkyl methylsiloxanes. Suitable alkyl methylsiloxanes include those having the general formula (I):

R - (SiMe₂-O-)ₓ SiMe₂R

where
- X =: 10-50
- Me =: CH₃ and
- R: is an alkyl group having an average alkyl chain length of at least C30.

It is important to note that suitable materials are usually made up of blends of terminal alkyl chain lengths. It is necessary for the purposes of this invention that the average terminal alkyl chain length is at least C30. Accordingly, a suitable structuring wax can have alkyl chain lengths below C30 but the average must be at least C30.

The waxes selected have long terminal alkyl chains which are extremely effective at structuring the composition. Pendant C30 and upwards species and C16 to C18 terminal alkyl waxes sometimes result in excessively soft solids which may fail to keep a stable structure following heat storage which results in an excessively thin composition. Similar difficulties are experienced when the average terminal alkyl chain length is below C30 e.g. C24 to C28.

A preferred alkylmethylsiloxane wax is known as GE wax SF1642 available from the General Electric Company in which the main alkyl species are C30, C32 and C34 with minor amounts of C26 and C28. The average terminal alkyl chain length is at least C30.

The structuring waxes have been found to exhibit a significant masking effect on the formulations to conceal deposition of actives and suspending agents. The alkylmethylsiloxane wax preferably has a melting point of greater than 45°C.

Preferably, the alkylmethylsiloxane wax is made up of C16 to C40 alkyl groups, more preferably C25 to C35.

Generally, such waxes will be made up of a blend of alkyl group chain lengths with the characteristics of the wax and hence its structuring effect being determined by the blend of chain lengths utilised.

Other structurant waxy materials which can be blended with the alkyl siloxane wax are high and low melting point waxes, gums, resins, polymers, starches and elastomers. High melting point waxes include insect and animal waxes such as beeswax and spermaceti; vegetable waxes such as carnauba, Uraturia, Japan wax, Douglas-fir bark wax, rice-bran wax, castor wax and bayberry wax; mineral waxes such as montan wax, peat wax, ozocerite and ceresin; petroleum waxes such as paraffin wax; synthetic waxes such as Filcher-Tropsch waxes, polyethylene waxes, chemically modified hydrocarbon waxes and substituted amide waxes. Examples of low melting point waxes include fatty acids, fatty alcohols, fatty acid esters and fatty acid amides having carbon chains of 3 to 30 carbon atoms. Particularly preferred low melting point waxes include stearyl alcohol, cetyl alcohol, myristyl alcohol and palmitic acid.

The wax structurant can be a single wax or blend of the aforementioned waxes selected to provide the desired melting point and skin feel characteristics. For instance, a combination of 6% SF1642 and 5% Castorwax MP80 is particularly effective and results in a wax structurant which shears at approximately 45°C.

### EXAMPLES

### Example 1

An anhydrous antiperspirant cream was produced having the following formulation:

| Ingredient | Function | % wt |
|---|---|---|
| AZAG (Powder) | Antiperspirant | 24.0 |
| Castorwax MP80 | Structurant | 5.0 |
| GE SF1642 (1229-425) | Silicone Wax & Deposit Masker | 6.0 |
| Volatile Silicone | Carrier Fluid | 44.0 |
| Non-Volatile Hydrocarbon (Albemarle Silkflo 364NF) | Deposit Masker | 14.0 |
| Talc (Suprafino) | Aid Dry Skin-Feel | 6.0 |
| Fragrance | | 1.0 |

The cream was made by adding Silkflo to Volatile Silicone starting the stirrer to half speed and heating to 66°C. The powders were drawn in and mixed for 5 minutes. When addition was complete, the mixture was reheated to 66°C. The waxes were premelted at 80°C and then added to the main vessel with stirring at full speed. The perfume was added and sheared for 5 minutes at 68 to 70°C.

### Comparative Data (Processing):

The abovementioned composition when processed at 68°C, sheared for 10 minutes and then filled at 55°C gave a composition having a viscosity of 220,000 cps. When the product was processed and filled at 68°C, and mixed as above for 10 minutes, the composition had a viscosity of 360,000 cps.

## Claims

1. A substantially anhydrous underarm cream composition suitable for topical application to the human skin, comprising an antiperspirant and/or deodorant agent, a non-volatile masking agent, and a structuring wax having the general formula:
R - (SiMe₂-O-)ₓ SiMe₂R
where
X = 10-50
Me = CH₃
wherein R is an alkyl group and the average terminal alkyl chain length is at least C30, **characterised in that** the composition has a cone penetration value of between approximately 10mm and 30mm where the cone has a diameter of 16.5mm and a weight of 102.5g.

2. An underarm composition as claimed in claim 1 **characterised in that** it comprises 3% to 20% by weight structuring wax.

3. An underarm composition as claimed in claim 2 **characterised in that** it comprises 5% to 15% by weight structuring wax.

4. An underarm composition as claimed in claims 1 to 3 **characterised in that** R is a C25 to C40 alkyl group.

5. An underarm composition as claimed in any of claims 1' to 4 **characterised in that** it further comprises a non-volatile masking oil.

6. An underarm composition as claimed in claim 5 **characterised in that** the non-volatile masking oil is selected from the group comprising non-volatile silicones and polyolefins or mixtures thereof.

7. An underarm composition as claimed in claim 6 **characterised in that** the polyolefin is polydecene.

8. An underarm composition as claimed in claim 7 further comprising a volatile carrier fluid.

9. An underarm composition as claimed in any of claims 1 to 8 **characterised in that** the volatile carrier fluid is a volatile silicone.

10. An underarm composition as claimed in any of claims 1 to 9 **characterised in that** the antiperspirant agent is an inorganic and/or organic salt of aluminium and zirconium or a mixture thereof.

11. An underarm composition as claimed in any of claims 1 to 10 **characterised in that** the structuring wax is blended with alkyl ester siloxane waxes, insect and animal waxes, fatty acids, fatty alcohols, fatty acid esters and fatty acid amides and mixtures thereof.

12. A substantially anhydrous cream antiperspirant composition comprising 5 to 30% by weight antiperspirant salt, 5% to 25% by weight of a non-volatile masking oil, 3% to 20% by weight of a structuring wax having the general formula:
R - (SiMe₂-O-)ₓ SiMe₂R
where
X = 10-50
Me = CH₃
R is an alkyl group and the average terminal alkyl chain length is at least C30 and 20 to 87% by weight of a volatile carrier fluid, **characterised in that** the composition has a cone penetration value of between approximately 10mm and 30mm where the cone has a diameter of 16.5mm and a weight of 102.5g.

## Patentansprüche

1. Im Wesentlichen wasserfreie Achselcremezusammensetzung, geeignet zur örtlichen Auftragung auf die menschliche Haut, umfassend ein schweißhemmendes und/oder desodorierendes Mittel, ein nicht-flüchtiges Maskierungsmittel und ein strukturierendes Wachs der allgemeinen Formel:
R - (SiMe₂-O-)ₓ SiMe₂R
worin
X = 10-50
Me = CH₃
wobei R eine Alkylgruppe darstellt und die mittlere Länge der endständigen Alkylketten mindestens C30 ist, **dadurch gekennzeichnet, dass** die Zusammensetzung einen Kegelpenetrationswert zwischen etwa 10 mm und 30 mm aufweist, wobei der Kegel einen Durchmesser von 16,5 mm und ein Gewicht von 102,5 g aufweist.

2. Achselzusammensetzung nach Anspruch 1, **dadurch gekennzeichnet, dass** sie 3% bis 20 Gewichtsprozent strukturierendes Wachs umfasst.

3. Achselzusammensetzung nach Anspruch 2, **dadurch gekennzeichnet, dass** sie 5% bis 15 Gewichtsprozent strukturierendes Wachs umfasst.

4. Achselzusammensetzung nach Ansprüchen 1 bis 3, **dadurch gekennzeichnet, dass** R eine C25- bis C40-Alkylgruppe darstellt.

5. Achselzusammensetzung nach einem der Ansprüche 1 bis 4, **dadurch gekennzeichnet, dass** sie weiterhin ein nicht-flüchtiges maskierendes Öl umfasst.

6. Achselzusammensetzung nach Anspruch 5, **dadurch gekennzeichnet, dass** das nicht-flüchtige maskierende Öl aus der Gruppe, umfassend nicht-flüchtige Silikone und Polyolefine oder Gemische davon, ausgewählt ist.

7. Achselzusammensetzung nach Anspruch 6, **dadurch gekennzeichnet, dass** das Polyolefin Polydecen darstellt.

8. Achselzusammensetzung nach Anspruch 7, die weiterhin ein flüchtiges Trägerfluid umfasst.

9. Achselzusammensetzung nach einem der Ansprüche 1 bis 8, **dadurch gekennzeichnet, dass** das flüchtige Trägerfluid ein flüchtiges Silikon darstellt.

10. Achselzusammensetzung nach einem der Ansprüche 1 bis 9, **dadurch gekennzeichnet, dass** das schweißhemmende Mittel ein anorganisches und/oder organisches Salz von Aluminium und Zirkonium oder ein Gemisch davon darstellt.

11. Achselzusammensetzung nach einem der Ansprüche 1 bis 10, **dadurch gekennzeichnet, dass** das strukturierende Wachs mit Alkylestersiloxanwachsen, Insekten- und Tierwachsen, Fettsäuren, Fettalkoholen, Fettsäureestern und Fettsäureamiden und Gemischen davon vermischt ist.

12. Im Wesentlichen wasserfreie, schweißhemmende Cremezusammensetzung, umfassend 5 bis 30 Gewichtsprozent schweißhemmendes Salz, 5% bis 25 Gewichtsprozent eines nichtflüchtigen, maskierenden Öls, 3% bis 20 Gewichtsprozent eines strukturierenden Wachses der allgemeinen Formel:
R - (SiMe₂-O-)ₓ SiMe₂R
worin
X = 10-50
Me = CH₃
R eine Alkylgruppe darstellt und die mittlere Länge der endständigen Alkylketten mindestens C30 ist, und 20 bis 87 Gewichtsprozent eines flüchtigen Trägerfluids, **dadurch gekennzeichnet, dass** die Zusammensetzung einen Kegelpenetrationswert zwischen etwa 10 mm und 30 mm aufweist, wobei der Kegel einen Durchmesser von 16,5 mm und ein Gewicht von 102,5 g aufweist.

## Revendications

1. Composition de crème pour les aisselles substantiellement anhydre appropriée pour une application topique que la peau humaine comprenant un agent antisudorifique et/ou un agent déodorant, un agent masquant non volatil et une cire structurante ayant la formule générale :
R-(Si Me₂-O-)ₓ Si Me₂ R dans laquelle
X = 10 - 50
Me = CH₃
dans laquelle R est un groupe alkyle et la longueur moyenne de la chaîne alkyle terminale est d'au moins C₃₀, **caractérisée en ce que** la composition à une valeur de pénétration de cône comprise entre approximativement 10 mm et 30 mm, dans laquelle le cône a un diamètre de 16,5 mm et un poids de 102,5 g.

2. Composition pour les aisselles selon la revendication 1, **caractérisée en ce qu'**elle comprend de 3 % à 20 % en poids d'une cire structurante.

3. Composition pour les aisselles selon la revendication 2, **caractérisée en ce qu'**elle comprend de 5 % à 15 % en poids d'une cire structurante.

4. Composition pour les aisselles selon les revendications 1 à 3, **caractérisée en ce que** R est un groupe alkyle en C₂₅ à C₄₀

5. Composition pour les aisselles selon l'une quelconque des revendications 1 à 4, **caractérisée en ce qu'**elle comprend en outre une huile non volatile masquante.

6. Composition pour les aisselles selon la revendication 5, **caractérisée en ce que** l'huile non volatile masquante est sélectionnée à partir du groupe comprenant les silicones non volatiles et les polyoléfines ou les mélanges de celles-ci.

7. Composition pour les aisselles selon la revendication 6, **caractérisée en ce que** la polyoléfine est du polydécène.

8. Composition selon la revendication 7, comprenant en outre un fluide volatil formant support.

9. Composition pour les aisselles selon l'une quelconque des revendications 1 à 8, **caractérisée en ce que** le fluide volatil formant support est une silicone volatile.

10. Composition pour les aisselles selon l'une quelconque des revendications 1 à 9, **caractérisée en ce que** l'agent antisudorifique est un sel organique ou inorganique d'aluminium et de zirconium ou un mélange de ceux-ci.

11. Composition pour les aisselles selon l'une quelconque des revendications 1 à 10, **caractérisée en ce que** la cire structurante est mélangée avec des cires alkyl ester siloxanes, des cires d'insectes et animales, des acides gras, des alcools gras, des esters d'acide gras et des amides d'acide gras et des mélanges de ceux-ci.

12. composition de crème antisudorifique substantiellement anhydre comprenant de 5 à 30 % en poids de sel antisudorifique, de 5 % à 25 % en poids d'une huile masquante non volatile, de 3 % à 20 % en poids d'une cire structurante ayant la formule générale suivante :
R-(Si Me₂-O-)ₓ Si Me₂ R
dans laquelle
X = 10 - 50
Me = CH₃
R est un groupe alkyle, et
la longueur moyenne de la chaîne alkyle terminale est d'au moins C₃₀, et de 20 à 87 % en poids d'un fluide volatil formant support, **caractérisée en ce que** la composition a une valeur de pénétration de cône comprise entre approximativement 10 mm et 30 mm alors que le cône a un diamètre de 16,5 mm et un poids de 102,5 g.
